# EUROPEAN PATENT SPECIFICATION

(11) **EP 1 913 402 B1**
(45) Date of publication and mention of the grant of the patent: **25.10.2017**
(21) Application number: 06780226.4
(22) Date of filing: 27.07.2006
(51) Int. Cl.: G01N 33/68, G01N 33/74

(54) **MONITORING OF CARDIAC NATRIURETIC PEPTIDES DURING DIAGNOSIS, MANAGMENT, AND TREATMENT OF CARDIAC DISEASES**
ÜBERWACHUNG KARDIALER NATRIURETISCHER PEPTIDE BEI DER DIAGNOSE UND BEHANDLUNG VON HERZKRANKHEITEN
SURVEILLANCE DES PEPTIDES NATRIURETIQUES CARDIAQUES DURANT LE DIAGNOSTIC, LA GESTION ET LE TRAITEMENT DES MALADIES CARDIAQUES

(30) Priority: 29.07.2005 US 703928 P
(43) Date of publication of application: 23.04.2008
(73) Proprietor: Koninklijke Philips N.V., 5656 AE Eindhoven (NL)
(72) Inventor: SEHER, Jens-Peter, D-70599 Stuttgart (DE); PLUSKAL, Malcolm, G., Acton, Massachusetts 01720 (US)
(74) Representative: de Haan, Poul Erik
(86) International application number: PCT/IB2006/052578
(87) International publication number: WO 2007/013041

(56) References cited:
- WO-A1-2005/019819
- US-A1- 2005 137 481
- GEGENHUBER ALFONS ET AL: "Time course of B-type natriuretic peptide (BNP) and N-terminal proBNP changes in patients with decompensated heart failure." CLINICAL CHEMISTRY FEB 2004, vol. 50, no. 2, February 2004 (2004-02), pages 454-456, XP002427032 ISSN: 0009-9147
- RADEMAKER M T ET AL: "CARDIAC NATRIURETIC PEPTIDES FOR CARDIAC HEALTH" CLINICAL SCIENCE, BIOCHEMICAL SOCIETY AND THE MEDICAL RESEARCH SOCIETY, LONDON,, GB, vol. 108, no. 1, January 2005 (2005-01), pages 23-36, XP009060567 ISSN: 0143-5221
- RICHARDS MARK ET AL: "NT-proBNP in heart failure: therapy decisions and monitoring." EUROPEAN JOURNAL OF HEART FAILURE : JOURNAL OF THE WORKING GROUP ON HEART FAILURE OF THE EUROPEAN SOCIETY OF CARDIOLOGY 15 MAR 2004, vol. 6, no. 3, 15 March 2004 (2004-03-15), pages 351-354, XP002427033 ISSN: 1388-9842
- BURGER ANDREW J ET AL: "Effect of nesiritide (B-type natriuretic peptide) and dobutamine on ventricular arrhythmias in the treatment of patients with acutely decompensated congestive heart failure: The PRECEDENT study." AMERICAN HEART JOURNAL, vol. 144, no. 6, December 2002 (2002-12), pages 1102-1108, XP002427034 ISSN: 0002-8703

## Description

### Background

The following relates to the medical diagnosis and treatment arts. It finds particular application in monitoring the diagnosis, management, and treatment of cardiac diseases based on levels of natriuretic peptides, and will be described with particular reference thereto. However, it also finds application in monitoring of cardiac diseases based on levels of various other cardiac markers.

According to the World Health Organization (WHO), the diagnosis for cardiac diseases is based on the presence of at least 2 of the following criteria: An electrocardiogram (ECG), patient history and serum cardiac markers. The 4 classical serum markers are: Total-CK (Creatine Kinase), CK-MB (Creatine Kinase MB isoenzyme), Myoglobin and cardiac Troponins. In the last years the natriuretic peptides have gained more and more interest as serum cardiac markers. A significant advance in the diagnosis and monitoring of cardiac diseases has been the development of blood automated and semi-automated assay tests that measure the level of a cardiac marker in a blood sample drawn from a patient known or suspected to be suffering from a cardiac disease. Some cardiac markers of interest include: atrial natriuretic peptide (ANP); N-terminal proANP (NT-proANP); brain natriuretic peptide (BNP); N-terminal pro-BNP (NT-proBNP); C-type natriuretic peptide (CNP); and so forth. These various cardiac markers are secreted by the patient's body during cardiac stress, and hence correlate with cardiac stress. During a heart attack, the level of cardiac markers typically increases sharply, and then decreases as the cardiac marker is utilized, metabolized or otherwise removed from the bloodstream. In the case of a congestive heart failure or other chronic cardiac stresses, a persistent level of such cardiac markers may be observed in the blood. Thus, the assayed level of cardiac marker is useful in detecting the presence of heart failure, determining its severity, and estimating prognosis. By monitoring cardiac marker levels in blood serum during treatment, the effectiveness of the treatment can be assessed.

Some of these cardiac markers have also been considered as possible therapeutic agents for treating cardiac diseases. Scios, Inc., for example, developed Natrecor^{®} (nesiritide), a recombinant form of human B-type natriuretic peptide (hBNP), which was approved by the Food and Drug Administration (FDA) in 2001 for the intravenous treatment of patients with certain types of acute heart failure. Synthetic therapeutic agents, such as nesiritide, which correspond to cardiac markers have shown substantial promise and demonstrated clinical success. In patients hospitalized with congestive heart failure, nesiritide reduces pulmonary capillary wedge pressure, pulmonary arterial pressure, right atrial pressure and systemic vascular resistance, all resulting in clinical improvement. Nesiritide has certain advantages over parental vasodilators (e.g. nitroprusside and nitroglycerin) and inotropic agents (e.g. dobutamine and milrinone) currently used in treating decompensated heart failure, being more effective and better tolerated, allowing patients to continue their usual medications while in the hospital, and not automatically requiring invasive monitoring. The terminal half-life of nesiritide in the bloodstream is 20 minutes, the onset of action is 15 min, and a fixed dose of nesiritide has a sustained effect over 24 hours.

Gegenhuber et al., in GEGENHUBER ALFONS ET AL: "Time course of 1-17 B-type natriuretic peptide (BNP) and N-terminal proBNP changes in patients with decompensated heart failure" CLINICAL CHEMISTRY FEB 2004, vol. 50, no. 2, February 2004 (2004-02), pages 454-456, describe administration of levosimendan (Simdax™; a small molecule calcium sensitizer) to patients with decompensated heart failure, and subsequent measurement of hemodynamics including pulmonary capillary wedge pressure (PCWP) and cardiac index. Changes in concentration of the cardiac biomarkers BNP and NT-proBNP were also measured.

However, a therapeutic agent which is a synthetic form of a cardiac marker or is otherwise mimetic of a cardiac marker typically interferes with the blood assay of the natural cardiac marker. For example, nesiritide is typically detected by the same blood assay techniques used to monitor the level of natural BNP. Accordingly, the BNP cardiac marker assay cannot separately monitor both the progression of heart failure (diagnostic aspect) and the efficiency of therapy using a synthetic form of the cardiac marker (kinetic aspect). It is desirable to monitoring both the kinetics of the administered therapeutic agent, and the progression of the underlying heart disease, in order to assess the effectiveness of the treatment.

### Brief Summary

According to one aspect, a cardiac monitoring method is disclosed. At least an inactive natriuretic peptide and an active natriuretic peptide are assayed in a biological sample taken from a patient. Both the inactive and active natriuretic peptides are produced in the patient responsive to cardiac stress. A therapeutic agent is administered to the patient. The administered therapeutic agent substantially interferes with the active natriuretic peptide assay but does not substantially interfere with the inactive natriuretic peptide assay. Kinetics of the administered therapeutic agent is determined based on at least the active natriuretic peptide assay. Cardiac diagnostic information is determined based on at least the inactive natriuretic peptide assay. The assay is performed both before and after administering the therapeutic; determination of the kinetics includes: determining a difference between (i) the active natriuretic peptide assay performed after the administering and (ii) the active natriuretic peptide assay performed before the administering to determine uptake of the administered therapeutic agent. The therapeutic agent includes a synthetic BNP, the active natriuretic peptide includes natural BNP, and the inactive natriuretic peptide includes NT-proBNP.

According to another aspect, a cardiac monitoring method is disclosed. A therapeutic agent is administered to a patient. A first cardiac marker assay is performed that is substantially affected by the therapeutic agent. A second cardiac marker assay is performed that is substantially not affected by the therapeutic agent. Kinetics of the administered therapeutic agent is determined based on at least the first cardiac marker assay. Cardiac diagnostic information is assessed based on at least the second cardiac marker assay. The first cardiac marker assay is repeated at least once to assess uptake of the therapeutic agent; the kinetics of the administered therapeutic agent is determined based on a change in the first cardiac marker between the repeated first cardiac marker assays; wherein the first cardiac marker includes a natural BNP, the second cardiac marker includes a natural NT-proBNP, and the therapeutic agent includes nesiritide.

According to another aspect, a cardiac monitoring apparatus is disclosed. A blood extraction device is adapted to extract a plurality of blood samples from a patient undergoing treatment including administration of a synthetic or mimetic form of an active natriuretic peptide. An assay device is adapted to perform a blood assay yielding at least (i) an active natriuretic peptide level of a naturally produced natriuretic peptide and the synthetic or mimetic peptide and (ii) an inactive natriuretic peptide level for each blood sample. An interpretation device outputs at least one of (i) kinetics of the administered synthetic or mimetic natriuretic peptide based on at least the first cardiac marker assay, and (ii) cardiac diagnosis information based on at least the second cardiac marker assay. The active natriuretic peptide includes BNP, the inactive natriuretic peptide includes NT-proBNP, and the administered synthetic or mimetic natriuretic peptide includes a synthetic BNP.

One advantage resides in providing both kinetic and diagnostic information during treatment of a cardiac disease using a therapeutic agent that interferes with cardiac marker monitoring.

Another advantage resides in facilitating treatment of cardiac disease using nesiritide or other natriuretic peptide therapeutic agents.

Another advantage resides in extracting both uptake of a therapeutic agent and cardiac diagnostic information from a common blood assay.

Numerous additional advantages and benefits will become apparent to those of ordinary skill in the art upon reading the following detailed description of the preferred embodiments.

### Brief Description of the Drawings

The invention may take form in various components and arrangements of components, and in various process operations and arrangements of process operations. The drawings are only for the purpose of illustrating preferred embodiments and are not to be construed as limiting the invention.
FIGURE 1 diagrammatically shows (i) one possible biochemical pathway for generating active BNP and inactive NT-proBNP, (ii) administered nesiritide, (iii) a first assays that measures a combination of the BNP and nesiritide levels, and (iv) a second assay that measures the level of NT-proBNP.
FIGURE 2 diagrammatically plots the marker and assay levels of FIGURE 1 for the case of a cardiac event such as a heart attack.
FIGURE 3 diagrammatically plots the marker and assay levels of FIGURE 1 for the case of treatment of chronic heart disease using a plurality of nesiritide treatments administered over a treatment period.
FIGURE 4 diagrammatically shows a suitable cardiac monitoring method for monitoring both nesiritide kinetics and cardiac disease progression.
FIGURE 5 diagrammatically shows a suitable cardiac monitoring apparatus for performing the cardiac monitoring method of FIGURE 4.

### Detailed Description of the Preferred Embodiments

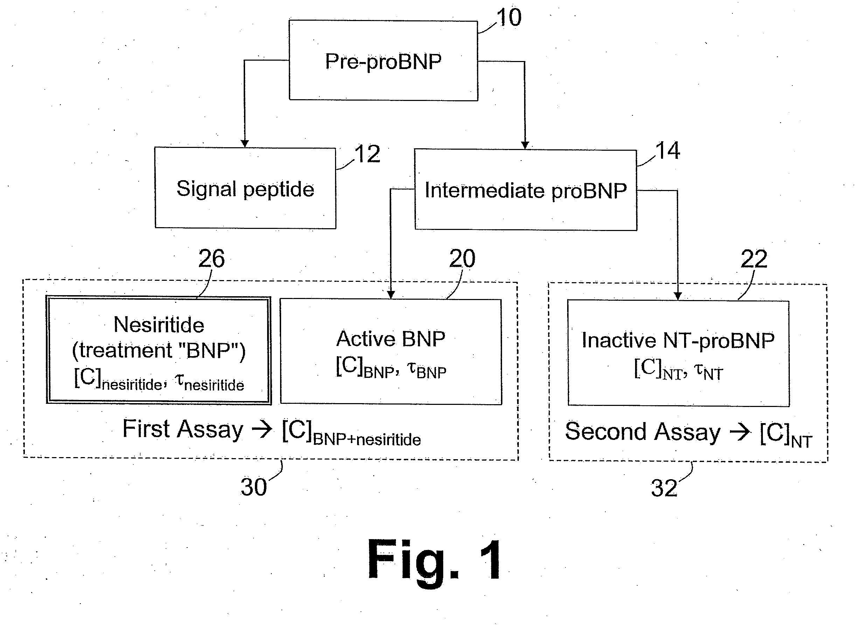

With reference to FIGURE 1, it is believed that the release or secretion of cardiac markers typically involves a series of biochemical reactions. Without being limited to any particular theory of operation, FIGURE 1 illustrates what is believed to be a typical series of biochemical reactions resulting in the release or secretion of active BNP from myocardial cells. A precursor pre-proBNP protein **10** typically having 134 amino acids is cleaved into a signal peptide **12** typically having 26 amino acids, and an intermediate proBNP protein **14** typically having 108 amino acids. The intermediate proBNP **14** may in turn be cleaved into a physiologically active form of BNP **20,** and a physiologically inactive form of NT-proBNP **22.**

The level of active BNP **20** in the blood is denoted herein as [C]_{BNP}. The level of inactive NT-proBNP **22** in the blood is denoted herein as [C]_{NT}. The levels of BNP **20** and NT-proBNP **22** are typically related. It is believed that this relationship is due to the production of BNP **20** and NT-proBNP **22** from a common biochemical pathway such that they are produced in equimolar amounts; however, the relationship between the levels of BNP **20** and NT-proBNP **22** may be due to other factors such as secretion responsive to a common cardiac stressor.

Although the blood serum levels of BNP **20** and NT-proBNP **22** are typically related, the physiologically inactive NT-proBNP **22** is relatively more stable and stays resident longer in the blood compared with the physiologically active BNP **20.** The terminal half-life of BNP **20** in the bloodstream is denoted herein as τ_{BNP}, and is believed to be typically of order of a few minutes to a few hours. In contrast, the terminal half-life of NT-proBNP **22** in the bloodstream, denoted herein as τ_{NT}, is typically substantially longer, and is believed to be about a month or longer.

The BNP **20** and NT-proBNP **22** cardiac markers are an example pair of related cardiac markers. It is expected that other pairs of cardiac markers may be similarly produced by related biochemical reactions or otherwise have related concentrations. Typically, if a common biochemical pathway or precursor molecule produces both an active natriuretic peptide or other cardiac marker and an inactive natriuretic peptide or other cardiac marker, then the terminal half-life of the inactive cardiac marker in the bloodstream will generally be substantially longer than the terminal half-life of the active cardiac marker in the bloodstream. It is expected that the inactive cardiac marker will typically have a terminal half-life at least ten times longer than the half-life of the active cardiac marker in the bloodstream, although the half-life difference may be less. For example, it is believed that ANP and NT-proANP are another related pair of active and inactive cardiac markers, respectively, derived from a common precursor molecule and hence generated in equimolar amounts. The terminal half-life of ANP in the bloodstream is believed to be substantially longer than the terminal half-life of NT-proANP in the bloodstream.

A physiologically active cardiac marker (BNP **20** in example FIGURE 1, or ANP as another example) is a candidate for use as a treatment. For example, nesiritide (available from Scios, Inc. under the trade name Natrecor^{®}) is a synthetic form of BNP that has been approved by the FDA for treatment of certain types of cardiac disease. Treatment agents that are synthetic ANP or mimetic of ANP are also of interest for treating arterial hypertension, congestive heart failure, and so forth.

Nesiritide **26** is used as a treatment agent in the example of FIGURE 1. Other treatment agents that are mimetic of active BNP **20** may also be used. A first blood assay **30** is performed to assay the level of BNP **20.** However, because nesiritide is typically detected by the same blood assay techniques used to monitor the level of natural BNP, the first assay **30** actually produces a level [C]_{BNP}+nesiritide which is indicative of a combination of the level [C]_{BNP} of natural BNP **20** in the blood serum and a level [C]_{nesiritide} of synthetic nesiritide **26** in the blood serum. Accordingly, the first assay **30** cannot provide, by itself, information on either the kinetics of the administered nesiritide **26** or the progression of the underlying heart disease indicated by the concentration [C]_{BNP} of natural BNP **20.**

Accordingly, a second blood serum assay **32** is performed to assay the level of NT-proBNP **22.** The second assay **32** is not substantially affected by the administered nesiritide **26;** accordingly, the second assay **32** can be used to determine cardiac diagnostic information. The first assay **30** can then be used to determine kinetics of the administered nesiritide **26.**

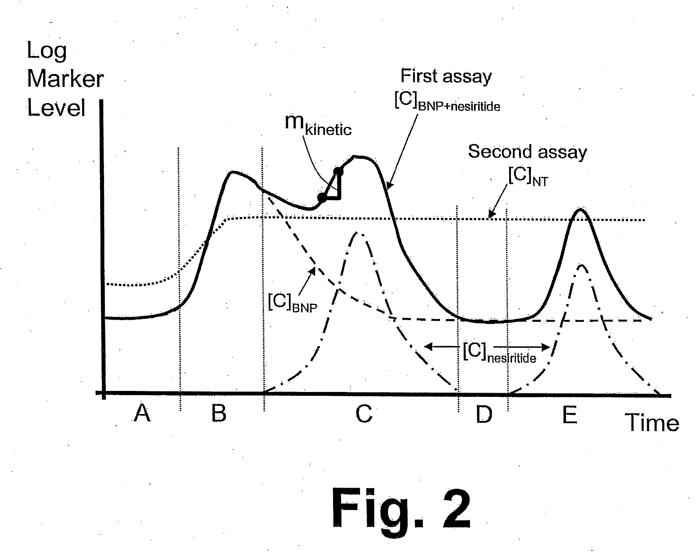

With reference to FIGURE 2, the monitoring provided by the first and second assays **30, 32** is illustrated in the case of a cardiac event such as a heart attack. Various regions of FIGURE 2 are denoted by letters "A", "B", "C", "D", and "E". The region "A" preceding the cardiac event exhibits persistent levels of BNP and NT-proBNP due to an underlying cardiac disease. At a transition from region "A" to region "B", the cardiac event occurs, causing cardiac stress. Responsive to the cardiac stress, the level [C]_{BNP} of BNP and the level [C]_{NT} of NT-proBNP both rise; however, by the end of region "B" the level of BNP begins to decrease due to its short terminal half-life in the bloodstream, and by the middle of the region "C" the level of BNP has returned to its persistent level indicative of the underlying cardiac disease, where it remains in regions "D" and "E". In contrast, the level of NT-proBNP remains elevated throughout regions "C", "D", and "E" due to its substantially longer half-life in the bloodstream. At the transition from region "B" to region "C" a dose of nesiritide administered, producing a transient level [C]_{nesiritide} of nesiritide in the blood.

The first assay **30** outputs a level [C]_{BNP+nesiritide} indicative of the combination of the level of nesiritide and the level of natural BNP. The second assay **32** outputs a level [C]_{NT} indicative of only the level of NT-proBNP in the blood. The second assay is suitably used to determine cardiac diagnostic information, such as to estimate the severity of the cardiac event.. The first assay **30** is suitably used to provide kinetics on the nesiritide treatment. For example, a difference or slope m_{kinetics} between two repetitions of the first assay **30** can be used to determine uptake of the nesiritide into the patient's bloodstream. In the region "D", the nesiritide dose has dissipated from the bloodstream, and the patient has returned to the persistent levels of BNP and NT-proBNP existing prior to the cardiac event. In the region "E", a follow-up dose of nesiritide is administered.

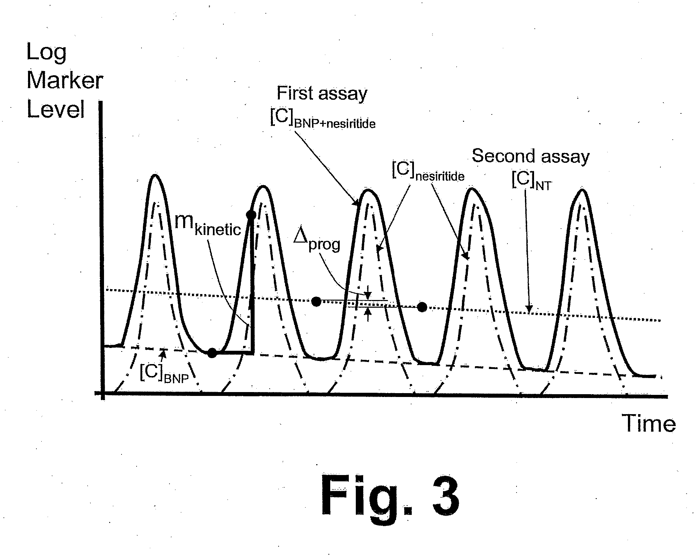

With reference to FIGURE 3, the monitoring provided by the first and second assays **30, 32** is illustrated in the case of the nesiritide being administered a plurality of times (e.g., daily, every third day, or so forth) over a treatment period (e.g., one-week, one-month, indefinitely). Each administration of nesiritide produces a transient level [C]_{nesiritide} in the bloodstream. The first assay **30** can be used to assess uptake of the nesiritide by, for example, taking a first repetition of the first assay **30** just before administering the nesiritide, and a second repetition of the first assay **30** a fixed time interval after administering the nesiritide. The difference in [C]_{BNP+nesiritide} level measured by the first and second repetitions is a measure of the uptake of the nesiritide. The terminal half-life of nesiritide in the bloodstream is typically about 20 minutes, while the onset of therapeutic action is about 15 minutes, and the therapeutic action is sustained over about 24 hours in typical cardiac patients. The first and second repetitions can be separated by, for example, about 2-10 minutes.

While the first assay **30** provides kinetic information about the administered treatment agent **26,** the second assay **32** provides diagnostic information about the progression of the cardiac disease. A difference Δ_{prog} between the level assayed using the second assay **32** on successive treatment dates shows, in the example FIGURE 3, that the persistent level of NT-proBNP is decreasing, presumably responsive to the nesiritide therapy, indicating improvement in symptoms of the underlying cardiac disease condition.

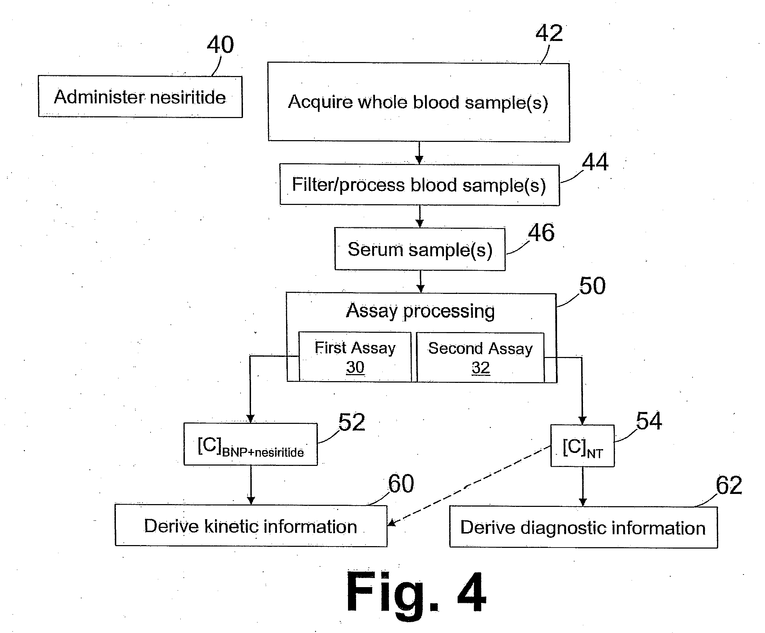

With reference to FIGURE 4, a suitable method for cardiac monitoring is described. In a treatment operation **40,** nesiritide or another cardiac marker-mimetic therapeutic agent is administered. One or more blood samples are drawn in an operation **42.** The blood samples can be drawn just prior to administering the nesiritide and/or just after administering the nesiritide. Typically, at least two blood samples are drawn to provide kinetic information about the nesiritide treatment. In a filtering process **44,** the white and red blood cells are substantially removed from the blood sample to produce a blood serum sample **46.** Assay processing **50** is performed to implement the first and second assays **30, 32.** It is to be appreciated that typically both the first and second assays **30, 32** can be performed using a single blood serum sample **46.** The first assay **30** provides the level [C]_{BNP+nesiritide} **52,** while the second assay **32** provides the level [C]_{NT} **54.** The level [C]_{BNP+nesiritide} **52** from two or more blood samples is used to determine kinetic information **60** about the administration **40** of nesiritide. The level [C]_{NT} **54** is used to determine diagnostic information **62** about the progression of the underlying cardiac disease.

In FIGURES 2 and 3, the kinetic information **60** is derived as a simple difference between the level [C]_{BNP}+nesiritide **52** measured from two successive blood samples. In other embodiments, a more elaborate derivation of kinetic information **60** may be performed. For example, because the levels [C]_{BNP} and [C]_{NT} are related, the level [C]_{BNP} may be estimated from the level [C]_{NT} assayed using the second assay **32.** Then, the level [C]_{BNP}+nesiritide **52** assayed using the first assay **30** may be corrected to remove the estimated level [C]_{BNP}, yielding the level [C]_{nesiritide}. In this approach, the level [C]_{nesiritide} of nesiritide may be assayable using only a single blood sample. In FIGURE 4, the contemplated use of the level [C]_{NT} in determining or refining the kinetic information is indicated by a connection arrow drawn in phantom connecting the level [C]_{NT} **54** with the kinetic information determination **60.**

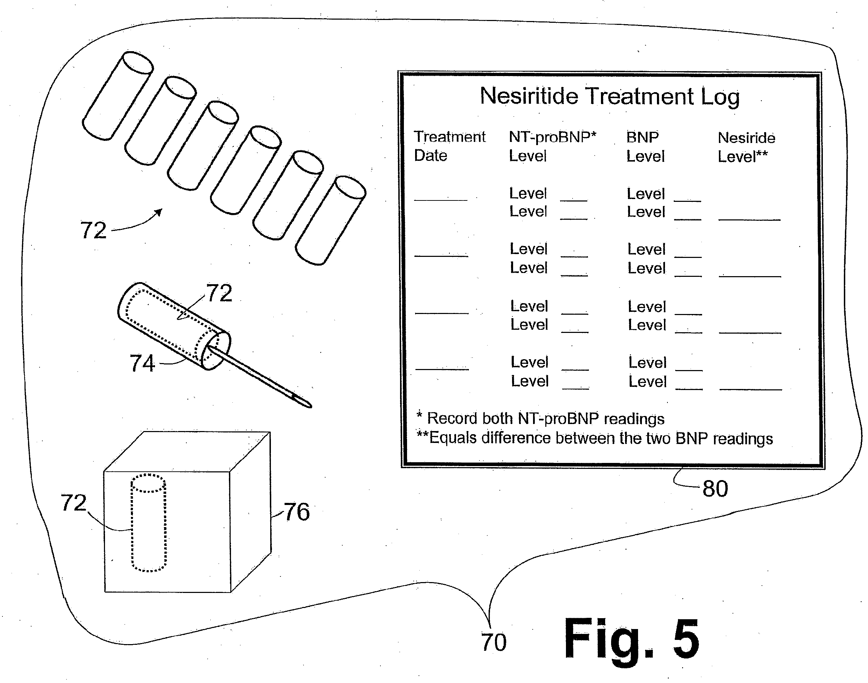

With reference to FIGURE 5, the described cardiac monitoring method advantageously employs existing techniques for assaying cardiac markers. Accordingly, a cardiac monitoring apparatus **70** is readily constructed, including a plurality of blood sample vials **72,** cartridges, or so forth, and a blood extraction device **74** (such as a hypodermic needle) adapted to sequentially extract blood samples into the blood sample vials **72.** An assay device **76** sequentially receives each filled blood sample vial **72** and performs standard BNP and NT-proBNP assays. The standard BNP assay corresponds to the first assay **30,** and actually yields a level [C]_{BNP+nesiritide} indicative of a combination of the active natural BNP **20** and synthetic nesiritide **26** levels. The standard NT-proBNP assay corresponds to the second assay **32** and yields the NT-proBNP level [C]_{NT}. An interpretation device **80** outputs the kinetics information and the diagnostic information.

In the illustrated cardiac monitoring apparatus **70,** the interpretation device **80** includes a "Nesiritide Treatment Log" card or display, on which for each date two sequential blood sample assays are recorded for each nesiritide treatment date over a treatment period. The two blood samples can be drawn before and a predetermined time after the nesiritide is administered, or can be drawn at two different predetermined times after the nesiritide is administered. By reading down the "NT-proBNP" column for successive treatment dates, the progression of the underlying cardiac disease can be ascertained. For example, if the recorded [C]_{NT} levels decrease over the course of the treatment period, this is an indication that the underlying cardiac disease is becoming less symptomatic. On the other hand, constant or increasing recorded [C]_{NT} levels suggest that the nesiritide treatment is ineffective. To derive the kinetic information on nesiritide uptake, the difference between the first assay **30** levels for the two blood samples drawn on a given treatment date is recorded. This difference is indicative of the uptake of nesiritide into the bloodstream.

Rather than a paper "Nesiritide Treatment Log" card **80,** an electronic log can be maintained. For example, the "Nesiritide Treatment Log" card **80** can be an electronic spreadsheet that includes a processor that automatically performs the difference computation to determine the nesiritide level. Moreover, such an electronic spreadsheet optionally includes a more complex kinetic information derivation, for example taking into account the NT-proBNP level [C]_{NT} assayed using the second assay 32. Optionally, the processor analyzes the assay results over time to provide alarms or warnings, proposed diagnoses, proposed nesiritide dosage adjustments, or so forth.

Because the disclosed cardiac monitoring methods and apparatuses employ standard blood assay techniques, the disclosed cardiac monitoring apparatus can be embodied as a near-patient monitoring device in a hospital, for example in an emergency room. Similarly, the disclosed cardiac monitoring apparatus can be embodied as a test station or apparatus located in a physician's office or laboratory. It is also contemplated to employ the disclosed cardiac monitoring apparatus as an in-home testing device for self-testing performed by the patient. For example, the patient can have the vials **72** and blood-drawing device **74** at home, and can draw blood samples at appropriate times. The blood samples are then sent to a laboratory which has the assay device **76** and interpretation device **80.** It is also contemplated to assay body fluids other than blood which may contain BNP, NT-proBNP, or other cardiac markers.

The illustrated methods and apparatuses using the combination of BNP and NT-proBNP assays to determine both kinetic and diagnostic information are examples. In other embodiments, for example, the first assay may monitor ANP, while the second assay may monitor NT-proANP. In other embodiments, the first assay may monitor CNP, while the second assay may monitor NT-proCNP. In other embodiments, the first assay may monitor DNP, while the second assay may monitor NT-proDNP. Still further, it is contemplated to monitor active and inactive cardiac markers derived from different biochemical pathways or precursor molecules. For example, monitoring of BNP and NT-proANP is contemplated. In this embodiment, BNP monitoring provides kinetic information on nesiritide uptake, while NT-proANP monitoring provides diagnostic information. Although BNP and NT-proANP are believed to be derived from separate biochemical pathways (and hence are not expected to be produced in equimolar amounts), both BNP and NT-proANP are believed to be produced in the patient predominantly due to cardiac stress; accordingly, monitoring both BNP and NT-proANP is expected to provide both kinetic and diagnostic information. Other assay combinations are also contemplated.

## Claims

1. A cardiac monitoring method comprising:
assaying at least an inactive natriuretic peptide **(22)** and an active natriuretic peptide **(20)** in a biological sample taken from a patient, both the inactive and active natriuretic peptides being produced in the patient responsive to cardiac stress;
wherein a therapeutic agent **(26)** has been administered to the patient, the administered therapeutic agent substantially interfering with the active natriuretic peptide assay but not substantially interfering with the inactive natriuretic peptide assay;
determining in the biological sample taken from the patient kinetics of the administered therapeutic agent based on at least the active natriuretic peptide assay; and
determining in the biological sample taken from the patient cardiac diagnostic information based on at least the inactive natriuretic peptide assay;
wherein the assaying is performed both before and after the administering, and the determining of kinetics includes:
determining a difference between (i) the active natriuretic peptide assay performed after the administering and (ii) the active natriuretic peptide assay performed before the administering to determine uptake of the administered therapeutic agent; and wherein the therapeutic agent includes a synthetic BNP, the active natriuretic peptide includes natural BNP and the inactive natriuretic peptide includes NT-proBNP.

2. The cardiac monitoring method as set forth in claim 1, wherein the assaying is performed a plurality of times, and the determining of kinetics includes:
determining a difference in the active natriuretic peptide assay performed the plurality of times, to determine uptake of the administered therapeutic agent.

3. The cardiac monitoring method as set forth in claim 1, wherein the administered therapeutic agent comprises nesiritide.

4. The cardiac monitoring method as set forth in claim 1, wherein a half-life of the inactive natriuretic peptide in the bloodstream is substantially longer than a half- life of the active natriuretic peptide in the bloodstream.

5. The cardiac monitoring method as set forth in claim 1, wherein a half-life of the inactive natriuretic peptide in the bloodstream is at least ten times longer than a half-life of the active natriuretic peptide in the bloodstream.

6. The cardiac monitoring method as set forth in claim 1, wherein the active natriuretic peptide and the inactive natriuretic peptide are produced by a common biochemical pathway or a common precursor molecule.

7. A cardiac monitoring method comprising:
performing on a biological sample taken from a patient a first cardiac marker assay that is substantially affected by a therapeutic agent which has been administered to the patient;
performing on a biological sample taken from the patient a second cardiac marker assay that is substantially not affected by the therapeutic agent;
determining kinetics of the administered therapeutic agent based on at least the first cardiac marker assay; and
assessing cardiac diagnostic information based on at least the second cardiac marker assay; and
repeating the performing of the first cardiac marker assay at least once to assess uptake of the therapeutic agent, the kinetics of the administered therapeutic agent being determined based on a change in the first cardiac marker between the repeated first cardiac marker assays;
wherein the first cardiac marker includes a natural BNP, the second cardiac marker includes a natural NT-proBNP, and the therapeutic agent includes nesiritide.

8. The cardiac monitoring method as set forth in claim 7, wherein the first cardiac marker and the second cardiac marker are produced by a common biochemical pathway or a common precursor molecule.

9. The cardiac monitoring method as set forth in claim 7, wherein the administering of the therapeutic agent is performed a plurality of times over a treatment period, and the performing of the second cardiac marker assay comprises:
repeating the performing of the second cardiac marker assay a plurality of times over the treatment period, the cardiac diagnostic information being based on increase or decrease of the assayed second cardiac marker over the treatment period.

10. A cardiac monitoring apparatus (70) comprising:
a blood extraction device (74) adapted to extract a plurality of blood samples from a patient undergoing treatment including administration of a synthetic or mimetic form of an active natriuretic peptide;
an assay device (76) adapted to perform a blood assay yielding at least (i) an active natriuretic peptide level of a naturally produced natriuretic peptide and the synthetic or mimetic natriuretic peptide in a first cardiac marker assay and (ii) an inactive natriuretic peptide level in a second cardiac marker assay for each blood sample; and
an interpretation device (80) adapted to output (i) kinetics of the administered synthetic or mimetic natriuretic peptide based on at least the first cardiac marker assay, and (ii) cardiac diagnosis information based on at least the second cardiac marker assay;
wherein the active natriuretic peptide includes BNP, the inactive natriuretic peptide includes NT-proBNP, and the administered synthetic or mimetic natriuretic peptide includes a synthetic BNP.

11. The cardiac monitoring apparatus as set forth in claim 10, wherein the interpretation device (80) comprises one of:
a card on which are recorded (i) level values of the blood assays and (ii) at least one computed value computed from one or more of the level values of one or more of the blood assays, and
an electronic spreadsheet in which are recorded level values of the blood assays, the electronic spreadsheet computing at least one computed value from one or more of the level values of one or more of the blood assays.

## Patentansprüche

1. Herzüberwachungsverfahren, umfassend:
Bestimmen von mindestens einem inaktiven natriuretischen Peptid (22) und einem aktiven natriuretischen Peptid (20) in einer einem Patienten entnommenen biologischen Probe, wobei sowohl das inaktive als auch das aktive natriuretische Peptid im Patienten in Reaktion auf kardialen Stress erzeugt werden;
wobei dem Patienten ein Therapeutikum (26) verabreicht wurde, wobei das verabreichte Therapeutikum die Bestimmung von aktivem natriuretischem Peptid erheblich beeinflusst, aber die Bestimmung von inaktivem natriuretischem Peptid nicht erheblich beeinflusst;
Ermitteln der Kinetik des verabreichten Therapeutikums in der dem Patienten entnommenen biologischen Probe basierend auf mindestens der Bestimmung von aktivem natriuretischem Peptid; und
Ermitteln von Herzdiagnoseinformationen in der dem Patienten entnommenen biologischen Probe basierend auf mindestens der Bestimmung von inaktivem natriuretischem Peptid;
wobei das Bestimmen sowohl vor als auch nach der Verabreichung durchgeführt wird, und wobei das Ermitteln der Kinetik Folgendes umfasst:
Ermitteln einer Differenz zwischen (i) der Bestimmung von aktivem natriuretischem Peptid nach der Verabreichung und (ii) der Bestimmung von aktivem natriuretischem Peptid vor der Verabreichung, um die Aufnahme des verabreichten Therapeutikums zu ermitteln; und wobei das Therapeutikum ein synthetisches BNP umfasst, das aktive natriuretische Peptid natürliches BNP umfasst und das inaktive natriuretische Peptid NT-proBNP umfasst.

2. Herzüberwachungsverfahren nach Anspruch 1, wobei die Bestimmung mehrmals durchgeführt wird und das Ermitteln der Kinetik Folgendes umfasst:
Ermitteln einer Differenz in der mehrmals durchgeführten Bestimmung von aktivem natriuretischem Peptid, um die Aufnahme des verabreichten Therapeutikums zu ermitteln.

3. Herzüberwachungsverfahren nach Anspruch 1, wobei das verabreichte Therapeutikum Nesiritid umfasst.

4. Herzüberwachungsverfahren nach Anspruch 1, wobei eine Halbwertszeit des inaktiven natriuretischen Peptids in dem Blutfluss wesentlich länger ist als eine Halbwertszeit des aktiven natriuretischen Peptids in dem Blutfluss.

5. Herzüberwachungsverfahren nach Anspruch 1, wobei eine Halbwertszeit des inaktiven natriuretischen Peptids in dem Blutfluss mindestens zehn Mal länger ist als eine Halbwertszeit des aktiven natriuretischen Peptids in dem Blutfluss.

6. Herzüberwachungsverfahren nach Anspruch 1, wobei das aktive natriuretische Peptid und das inaktive natriuretische Peptid durch einen gemeinsamen biochemischen Pfad oder ein gemeinsames Vorläufermolekül erzeugt werden.

7. Herzüberwachungsverfahren, umfassend:
Durchführen einer Bestimmung eines ersten Herzmarkers für eine einem Patienten entnommenen biologischen Probe, wobei die Bestimmung erheblich durch ein dem Patienten verabreichtes Therapeutikum beeinflusst wird;
Durchführen einer Bestimmung eines zweiten Herzmarkers für eine einem Patienten entnommenen biologischen Probe, wobei die Bestimmung nicht erheblich durch ein dem Patienten verabreichtes Therapeutikum beeinflusst wird;
Ermitteln der Kinetik des verabreichten Therapeutikums basierend auf mindestens der Bestimmung des ersten Herzmarkers; und
Beurteilen von Herzdiagnoseinformationen basierend auf mindestens der Bestimmung des zweiten Herzmarkers; und
Wiederholen des Durchführens der Bestimmung des ersten Herzmarkers mindestens ein Mal, um die Aufnahme des Therapeutikums zu beurteilen, wobei die Kinetik des verabreichten Therapeutikums basierend auf einer Änderung in dem ersten Herzmarker zwischen den wiederholten Bestimmungen des ersten Herzmarkers ermittelt wird;
wobei der erste Herzmarker ein natürliches BNP umfasst, der zweite Herzmarker ein natürliches NT-proBNP umfasst und das Therapeutikum Nesiritid umfasst.

8. Herzüberwachungsverfahren nach Anspruch 7, wobei der erste Herzmarker und der zweite Herzmarker durch einen gemeinsamen biochemischen Pfad oder ein gemeinsames Vorläufermolekül erzeugt werden.

9. Herzüberwachungsverfahren nach Anspruch 7, wobei das Verabreichen des Therapeutikums mehrere Male während eines Behandlungszeitraums erfolgt und das Durchführen der Bestimmung des zweiten Herzmarkers Folgendes umfasst:
Wiederholen des Durchführens der Bestimmung des zweiten Herzmarkers mehrere Male während des Behandlungszeitraums, wobei die Herzdiagnoseinformationen auf einem Anstieg oder einer Abnahme des bestimmten zweiten Herzmarkers über den Behandlungszeitraum basieren.

10. Herzüberwachungsgerät (70), umfassend:
eine Blutentnahmevorrichtung (74), die dafür ausgelegt ist, einem Patienten, der einer Behandlung umfassend die Verabreichung einer synthetischen oder mimetischen Form eines aktiven natriuretischen Peptids unterzogen wird, eine Vielzahl von Blutproben zu entnehmen;
eine Bestimmungsvorrichtung (76), die dafür ausgelegt ist, eine Blutanalyse durchzuführen, mit der mindestens (i) der aktive natriuretische Peptidspiegel eines natürlich erzeugten natriuretischen Peptids und des synthetischen oder mimetischen natriuretischen Peptids in einer Bestimmung des ersten Herzmarkers und (ii) ein inaktiver natriuretischer Peptidspiegel in einer Bestimmung des zweiten Herzmarkers für jede Blutprobe erlangt wird; und
eine Interpretationsvorrichtung (80), die dafür ausgelegt ist, Folgendes auszugeben: (i) die Kinetik des verabreichten synthetischen oder mimetischen natriuretischen Peptids basierend auf mindestens der Bestimmung des ersten Herzmarkers, und (ii) Herzdiagnoseinformationen basierend auf mindestens der Bestimmung des zweiten Herzmarkers;
wobei das aktive natriuretische Peptid BNP umfasst, das inaktive natriuretische Peptid NT-proBNP umfasst und das verabreichte synthetische oder mimetische natriuretische Peptid ein synthetisches BNP umfasst.

11. Herzüberwachungsgerät nach Anspruch 10, wobei die Interpretationsvorrichtung (80) eines von Folgendem umfasst:
eine Karte, auf der aufgezeichnet werden: (i) die mit den Blutanalysen ermittelten Spiegelwerte und (ii) mindestens ein berechneter Wert, der anhand von einem oder mehreren der Spiegelwerte der einen oder mehreren Blutanalysen berechnet wurde, und
ein elektronisches Tabellenblatt, in dem die mit der Blutanalyse ermittelten Spiegelwerte aufgezeichnet werden, wobei das elektronische Tabellenblatt mindestens einen berechneten Wert anhand von einem oder mehreren der Spiegelwerte von einer oder mehreren der Blutanalysen berechnet.

## Revendications

1. Procédé de surveillance cardiaque comprenant :
le test d'au moins un peptide natriurétique inactif (22) et d'un peptide natriurétique actif (20) dans un échantillon biologique prélevé sur un patient, les peptides natriurétiques inactif et actif étant tous deux produits par le patient en réponse à un stress cardiaque ;
dans lequel un agent thérapeutique (26) a été administré au patient, l'agent thérapeutique administré interférant sensiblement avec le test de peptide natriurétique actif mais n'interférant pas sensiblement avec le test de peptide natriurétique inactif ;
la détermination dans l'échantillon biologique prélevé sur le patient de la cinétique de l'agent thérapeutique administré sur la base au moins du test de peptide natriurétique actif ; et
la détermination dans l'échantillon biologique prélevé sur le patient d'informations de diagnostic cardiaque basées sur au moins le test de peptide natriurétique inactif ;
dans lequel le test est réalisé à la fois avant et après l'administration et la détermination de la cinétique comprend :
la détermination d'une différence entre (i) le test de peptide natriurétique actif réalisé après l'administration et (ii) le test de peptide natriurétique actif réalisé avant l'administration pour déterminer la prise de l'agent thérapeutique administré ; et dans lequel l'agent thérapeutique comprend un BNP synthétique, le peptide natriurétique actif comprend un BNP naturel et le peptide natriurétique inactif comprend un NT-proBNP.

2. Procédé de surveillance cardiaque selon la revendication 1, dans lequel le test est réalisé plusieurs fois, et la détermination de la cinétique comprend :
la détermination d'une différence dans le test de peptide natriurétique actif réalisé plusieurs fois, pour déterminer la prise de l'agent thérapeutique administré.

3. Procédé de surveillance cardiaque selon la revendication 1, dans lequel l'agent thérapeutique administré comprend du nésiritide.

4. Procédé de surveillance cardiaque selon la revendication 1, dans lequel une demi-vie du peptide natriurétique inactif dans le flux sanguin est sensiblement plus longue qu'une demi-vie du peptide natriurétique actif dans le flux sanguin.

5. Procédé de surveillance cardiaque selon la revendication 1, dans lequel une demi-vie du peptide natriurétique inactif dans le flux sanguin est au moins dix fois plus longue qu'une demi-vie du peptide natriurétique actif dans le flux sanguin.

6. Procédé de surveillance cardiaque selon la revendication 1, dans lequel le peptide natriurétique actif et le peptide natriurétique inactif sont produits par un chemin biochimique commun ou une molécule précurseur commune.

7. Procédé de surveillance cardiaque comprenant :
la réalisation sur un échantillon biologique prélevé sur un patient d'un premier test de marqueur cardiaque qui est sensiblement affecté par un agent thérapeutique qui a été administré au patient ;
la réalisation sur un échantillon biologique prélevé sur le patient d'un second test de marqueur cardiaque qui est sensiblement non affecté par l'agent thérapeutique ;
la détermination de la cinétique de l'agent thérapeutique administré sur la base d'au moins le premier test de marqueur cardiaque ; et
l'évaluation des informations de diagnostic cardiaque sur la base d'au moins le second test de marqueur cardiaque ; et
la répétition de la réalisation du premier test de marqueur cardiaque au moins une fois pour évaluer la prise de l'agent thérapeutique, la cinétique de l'agent thérapeutique administré étant déterminée sur la base d'un changement du premier marqueur cardiaque entre les premiers tests de marqueur cardiaque répétés ;
dans lequel le premier marqueur cardiaque comprend un BNP naturel, le second marqueur cardiaque comprend un NT-proBNP naturel et l'agent thérapeutique comprend du nésiritide.

8. Procédé de surveillance cardiaque selon la revendication 7, dans lequel le premier marqueur cardiaque et le second marqueur cardiaque sont produits par un chemin biochimique commun ou une molécule précurseur commune.

9. Procédé de surveillance cardiaque selon la revendication 7, dans lequel l'administration de l'agent thérapeutique est réalisée plusieurs fois sur une période de traitement, et la réalisation du second test de marqueur cardiaque comprend :
la répétition de la réalisation du second test de marqueur cardiaque plusieurs fois sur la période de traitement, les informations de diagnostic cardiaque étant basées sur une augmentation ou une diminution du second marqueur cardiaque testé sur la période de traitement.

10. Appareil de surveillance cardiaque (70) comprenant :
un dispositif de prise de sang (74) adapté pour extraire une pluralité de prélèvements sanguins d'un patient subissant un traitement comprenant l'administration d'une forme synthétique ou mimétique d'un peptide natriurétique actif ;
un dispositif de test (76) adapté pour réaliser un test sanguin donnant au moins (i) un niveau de peptide natriurétique actif d'un peptide natriurétique produit naturellement et le peptide natriurétique synthétique ou mimétique dans un premier essai de marqueur cardiaque et (ii) un niveau de peptide natriurétique inactif dans un second test de marqueur cardiaque pour chaque prélèvement sanguin ; et
un dispositif d'interprétation (80) adapté pour émettre (i) la cinétique du peptide natriurétique synthétique ou mimétique administré sur la base d'au moins le premier test de marqueur cardiaque et (ii) des informations de diagnostic cardiaque basées sur au moins le second test de marqueur cardiaque ;
dans lequel le peptide natriurétique actif comprend un BNP, le peptide natriurétique inactif comprend un NT-proBNP, et le peptide natriurétique synthétique ou mimétique administré comprend un BNP synthétique.

11. Appareil de surveillance cardiaque selon la revendication 10, dans lequel le dispositif d'interprétation (80) comprend un élément parmi :
une carte sur laquelle sont enregistrées (i) des valeurs de niveau des tests sanguins et (ii) au moins une valeur calculée à partir d'une ou plusieurs des valeurs de niveau d'un ou plusieurs de tests sanguins, et
un tableur électronique dans lequel sont enregistrées des valeurs de niveau des tests sanguins, le tableur électronique calculant au moins une valeur calculée à partir d'une ou plusieurs des valeurs de niveau d'un ou plusieurs de tests sanguins.
